(19) Europäisches Patentamt
European Patent Office
Office européen des brevets

(11) EP 0 642 488 B1

(12) **EUROPEAN PATENT SPECIFICATION**

(45) Date of publication and mention of the grant of the patent:
**09.04.1997 Bulletin 1997/15**

(21) Application number: **93912813.8**

(22) Date of filing: **26.05.1993**

(51) Int Cl.6: **C07C 45/50**, C07C 45/85, C07C 45/78, C07C 45/80

(86) International application number:
**PCT/EP93/01340**

(87) International publication number:
**WO 93/24437 (09.12.1993 Gazette 1993/29)**

(54) **CATALYST REGENERATION**

KATALYSATORREGENERIERUNG

REGENERATION DE CATALYSEUR

(84) Designated Contracting States:
**BE DE FR GB IT NL SE**

(30) Priority: **29.05.1992 US 891341**
**25.03.1993 GB 9306190**

(43) Date of publication of application:
**15.03.1995 Bulletin 1995/11**

(73) Proprietor: **EXXON CHEMICAL PATENTS INC.**
**Linden New Jersey 07036 (US)**

(72) Inventors:
• **DE RIJKE, Jan, Martin**
**B-2540 Hove (BE)**

• **VAN DRIESSCHE, Eddy**
**B-9900 Eekloo (BE)**
• **HANIN, Jean, Alexandre, Andre**
**B-1330 Rixensart (BE)**

(74) Representative: **Bawden, Peter Charles et al**
**EXXON CHEMICAL LIMITED**
**Exxon Chemical Technology Centre**
**PO Box 1**
**Abingdon Oxfordshire OX13 6BB (GB)**

(56) References cited:
EP-A- 0 183 546       FR-A- 2 235 087
GB-A- 2 055 371       US-A- 3 725 534

**EP 0 642 488 B1**

**Description**

This invention relates to the oxo process and, in particular, to the catalyst cycle of the oxo process. That is, it relates to a method for removing metal-containing catalyst residues, for example, cobalt-containing residues, from crude oxo products and recovering therefrom catalyst residues in a form suitable for recycle to the oxo reaction.

Oxo reactions (also referred to as hydroformylation reactions) involve the preparation of oxygenated organic compounds by the reaction of a carbon monoxide and hydrogen mixture ("syn gas") with carbon compounds containing olefinic unsaturation. The reaction is performed in the presence of a hydroformylation catalyst and results in the formation of a compound, for example, an aldehyde, which has one more carbon atom in its molecular structure than the starting olefinic feedstock. By way of example, higher alcohols may be produced by hydroformylation of commercial $C_5$ to $C_{12}$ olefin fractions to a $C_6$ to $C_{13}$ aldehyde-containing oxonation product, which on hydrogenation and distillation yields the corresponding alcohol. The crude product of the hydroformylation reaction will contain catalyst, aldehydes, alcohols, unreacted feedstock, syn gas and by-products.

Before further processing of the crude product is possible, it is necessary to remove the catalyst therefrom. One conventional method of removing cobalt values (that is, cobalt in any one of its possible oxidation states) from such a crude product is to treat the product with an alkali or acid wash technique; however, this uses expensive raw materials and, moreover, it is difficult to remove essentially all traces of cobalt from the resulting water wash streams before those may be discharged into water courses. Another such conventional method involves the oxidation of the cobalt catalytic species followed by extraction as a salt in aqueous solution.

U.S. Patent No. 3725534 describes a method for recovery of cobalt values from crude oxo product in which the liquid oxo product containing substantially all the cobalt values in the form of dissolved cobalt compounds is mixed with an aqueous acid, the cobalt materials being converted to water-soluble salts of the acid. After it has been combined with a promotor, for example, an alkali or alkaline earth metal salt of a weak acid, the separated aqueous phase is introduced into a preformer containing a preforming catalyst, for example, a noble metal catalyst. The effluent is then treated, preferably with a strong mineral acid, to convert complex cobalt carbonyl compounds to volatile hydrocobalt carbonyl, which is stripped out of the acidified effluent by an inert gas or syn gas, absorbed in an organic solvent and fed to the oxonation reactor.

In DE-A-2244373 and U.S. Patent No. 3957684, there are described methods in which aqueous solutions of cobalt salts are treated with syn gas in the presence of a substantially immiscible organic solvent containing cobalt carbonyl and/or hydrocobalt carbonyl under conditions ensuring turbulent flow. The preferred solvent is n-butanol.

In GB-A-2055371 there is described a two-stage method for recovering cobalt from an oxo product, the first stage comprising contacting the oxo product with an aqueous cobalt salt to form a $Co[Co(CO)_4]_2$-containing product which is recycled via a preformer to the oxo reactor, and completing removal of cobalt in a second stage using oxygen and an aqueous acid, the aqueous reaction product being used as the aqueous cobalt salt in the first stage. Preforming may be effected in the presence of syn gas and an organic solvent.

In U.S. Patent No. 4625067 there is disclosed a method of removing cobalt values from the crude product of a cobalt-catalysed hydroformylation reaction by contacting the crude product with a stream of stripping gas to entrain volatile cobalt compounds, said contacting being performed in the presence of water or an aqueous acid to dissolve those cobalt values not entrained in the gas under the conditions of temperature and pressure employed for said contacting, the aqueous phase subsequently being separated from the organic hydroformylation reaction product. The separated cobalt-containing aqueous phase is concentrated in a flash unit, and the concentrate recycled either directly to the oxo reactor or passed to a preformer unit where volatile cobalt carbonyls are removed by means of a flow of gas, preferably syn gas, injected into the product leaving the oxo reactor and recycled with unreacted syn gas indirectly to the reactor.

In principle, all cobalt values are removed from the aqueous treatment phase by the procedures described in the above-mentioned patents and recycled to the oxonation reactor. In practice, however, some cobalt remains in the aqueous phase and presents disposal problems. Further, the aqueous phase is acidic, usually containing organic contaminants, formic acid, and possibly formates, and disposal of such aqueous material presents problems whether or not it contains cobalt values.

Additionally, when the olefin being hydroformylated is of lower molecular weight, more especially from $C_5$ to $C_8$, any saturated, and therefore unreactive, hydrocarbons contained in the feedstock as impurity have a sufficient similarity in volatility to be entrained together with the volatile cobalt carbonyls recycled to the hydroformylation reactor. The same is true of any less reactive lower molecular weight olefinic species, especially $C_5$ to $C_8$ materials, and hence, as reaction proceeds, the concentration of unreactive species in the feedstock builds up to unacceptable proportions.

In general, in the above described procedures and in many others, stripping is carried out as an integrated part of the oxonation, in the presence of a reactant or reaction product of the process. Any changes in procedure for recovery of the cobalt values have an effect on, and may require change in, the manufacturing process itself, which is undesirable.

In one aspect of the present invention, there is provided a cobalt value recovery process with no inherent aqueous effluent stream and in which any intermittent or optional aqueous effluent may be substantially free from cobalt.

In a second aspect of the invention, there is provided an oxonation process in which cobalt catalyst regeneration may be carried out largely independently of the main oxonation process.

In an advantageous embodiment of the second aspect, catalyst regeneration is carried out in the absence both of olefinic feedstock to the oxonation process and of the oxonation product.

In a third aspect, there is provided a cobalt-catalysed oxonation process in which the regeneration of catalyst is effected using only reactants that may be, and preferably are, used in the oxonation process itself.

The present invention provides a method for recovering cobalt values from a liquid organic phase containing them, the method comprising contacting the organic phase with an aqueous phase having a pH of at most 7 that is substantially immiscible with the liquid organic phase and capable of extracting cobalt values from the organic phase, and separating the resulting cobalt-containing aqueous phase and cobalt-depleted organic phase,

contacting the separated cobalt-containing aqueous phase with syn gas and an organic solvent for dicobalt carbonyl and hydrocobalt carbonyl, the organic solvent being substantially immiscible with the cobalt-containing aqueous phase, and recovering from the aqueous phase and the organic phase so obtained volatile cobalt compounds, separating the resulting organic and aqueous phases, and returning the resulting organic phase to form the said organic solvent,
and returning the resulting aqueous phase to form the said aqueous phase having a pH of at most 7.

(For the sake of clarity, organic phases that are in liquid form are referred to throughout the specification as "liquid organic phases". It will be appreciated that the aqueous phases referred to herein are all in the liquid phase.)

The liquid organic phase containing cobalt values may be the crude product of a cobalt-catalysed oxo reaction, and will be referred to as such for simplicity in the following description.

The contact between the crude oxo product and the aqueous phase may be carried out in the presence or the absence of air. If this step in the method, the "demetalling" step, is carried out in the presence of air, it is preferred to concentrate the separated cobalt-containing aqueous phase before contacting it with syn gas and organic solvent, yielding a concentrate and a substantially cobalt-free aqueous phase. (Preferred concentration procedures are described in more detail below).

Advantageously, the cobalt-depleted organic phase resulting from contact of the organic phase containing cobalt values and the aqueous phase of pH at most 7 is washed with a substantially cobalt-free aqueous phase and, if it is or comprises an oxonation product, may preferably be fed to a hydrogenation reactor. The substantially cobalt-free aqueous phase used for this purpose is advantageously the substantially cobalt-free product of concentrating the separated cobalt-containing aqueous phase.

If demetalling is carried out in the presence of air, initial contact of the cobalt-containing aqueous phase, preferably after concentration, with syn gas and organic solvent preferably takes place in a preformer reactor, to be described in more detail below, and recovery of the resulting volatile cobalt compounds from the aqueous phase is advantageously effected by passing the reaction mixture from the preformer to a stripping zone into which additional stripping gas, advantageously syn gas, is fed, the volatile cobalt compounds being taken off as overhead with the liquid organic and aqueous phases recovered as bottoms product for subsequent separation.

If airless demetalling is employed, the preferred procedure differs slightly. In this case the cobalt-containing aqueous phase is preferably not concentrated before its first contact with the organic solvent but instead is passed first to a stripping zone, into which a composition including organic solvent is also being passed, as well as stripping gas, advantageously syn gas. As before, volatile cobalt compounds are taken off as overhead from the stripper and liquid organic and aqueous phases, now substantially depleted of volatile cobalt compounds, recovered as bottoms for separation, the resulting separated organic phase then being recycled to form the organic solvent.

After separation of aqueous and organic phases, the aqueous phase is advantageously divided, a part advantageously being concentrated, and another part used as the source of the aqueous phase having a pH of at most 7 for the demetalling step. After concentration, if employed, the cobalt-containing aqueous phase is passed to a preforming reactor, together with syn gas and organic solvent from the separator, the resulting product forming the composition passed to the stripping zone.

The aqueous phase contacted with the crude oxo product for demetalling may be water or an aqueous acid, which may already contain cobalt salts. Cobalt in the crude oxo product is taken up in the form of salts in the aqueous phase.

In the case where the aqueous phase is water (rather than aqueous acid), then cobalt may be transformed into $Co[Co(CO)_4]_2$ but, because of the presence of formic acid, produced in the oxo reaction and present in the oxo product, in the presence of air all cobalt present will be transformed into cobalt formate.

In the absence of air, but in the presence of $Co^{2+}$, hydrocobalt carbonyl will be transformed into $Co[Co(CO)_4]_2$. Because of the presence of formic acid, a proportion of the $Co[Co(CO)_4]_2$ is transformed into cobalt formate or hydrocobalt carbonyl.

Preferably, however, rather than water, both in the presence or absence of air, a solution of a light organic acid is used as the aqueous phase in demetalling, for example, an acetic or formic acid solution. It is especially preferred that the aqueous phase should comprise dilute formic acid, for example, at concentrations of from 1 to 5, especially from 1 to 2, wt %. Preferably, the water or aqueous acid is used in an amount of from 5 to 20 wt. % based on the amount of crude product. It is particularly preferred to use aqueous formic acid as the aqueous phase required to be present in the demetalling step, as formic acid is one of the organic compounds produced in the hydroformylation reaction and its associated secondary reactions to which this invention primarily relates.

The contact between the cobalt-containing aqueous phase, syn gas, and organic solvent to form cobalt carbonyls may be carried out in the preformer in the presence or absence of a suitable catalyst.

If a catalyst is present, it is advantageously a noble metal, especially gold, platinum or, preferably, palladium, either in the form of a slurry or, preferably, in a fixed bed. Advantageously, the active catalyst is embedded on a solid support, e.g., carbon or alumina. A 1 to 2% palladium-carbon fixed bed catalyst is preferred.

If no catalyst is present, the preformer is preferably one designed to maximize contact between the two liquid phases and the gas. As an example of a suitable preformer, there may be mentioned a tubular reactor having a high length: diameter ratio, for example from 100 to 100 000, advantageously from 1000 to 100 000, under conditions that result in at least partially turbulent flow, advantageously with a Reynolds Number much higher than 1600, and preferably above 3000. Optionally, but advantageously, at least one of the phases contains cobalt carbonyl and/or hydrocobalt carbonyl as initiator, thereby minimizing or avoiding an induction period for reaction.

As indicated above, the volatile cobalt carbonyls are advantageously recovered from the aqueous phase and the organic liquid phase in a stripping step in which the said phases are contacted with a stripping gas, the volatile cobalt compounds being entrained in the stripping gas.

As the stripping gas in the stripping step there is preferably used syn gas, the particular proportions of the carbon monoxide and hydrogen in the syn gas being adjusted to suit the reaction system. The stripping gas may, in principle, be any gas that does not react in an undesired manner with the cobalt compounds to be entrained therein, for example, nitrogen or a light saturated hydrocarbon such as propane or butane, but syn gas is preferred.

Advantageously the volume ratio of stripping gas to liquid phases at the applied conditions is from 20:1 to 250:1, preferably from 50:1 to 125:1. Higher ratios may be used, with good effect, but high ratios may be detrimental economically. The limiting lower value of the ratio, it will be appreciated, will be reached when there is insufficient stripping gas flow to achieve the desired degree of cobalt volatiles removal. Similarly any flow rate of stripping gas may be used, provided that it is sufficient to give the desired entrainment of cobalt volatiles. The liquid (organic and aqueous) phases are preferably well dispersed to give good contact between the liquids. It is preferred, too, that the stripper unit in which the stripping step is performed should include inert solid surfaces, for example, trays or packing material, to facilitate contact between the liquid and gas phases.

The volatile cobalt compounds recovered by the process of the invention are advantageously utilized in the oxonation reaction. Preferably, the stripping gas containing the volatile compounds is contacted with olefin being fed to the oxo reactor, the stripping gas advantageously being passed in countercurrent to the olefinic feed. The volatile cobalt compounds entrained in the stripping gas dissolve in the olefinic feed and are thus returned to the oxo reaction zone. The stripping gas may then either be purged or recycled. It will be appreciated therefore that syn gas is the preferred stripping gas because its subsequent recycle contact with the olefinic feedstock, or use in the preformer or stripper, will not cause the introduction of undesirable materials into the oxo reactor. Under the conditions employed, substantially all volatile cobalt compounds will be entrained by the stripping gas and none retained in the bottoms product. Similarly, substantially no water or organic solvent will be carried over by the stripping gas (syn gas) and introduced into the olefinic feed. If necessary the cobalt-containing stripping gas may be cooled prior to contact with the olefinic feed, and condensed water refluxed into the stripper, or fed to the preformer as a source of volatile cobalt as initiator.

As indicated above, the aqueous phase containing cobalt values is advantageously concentrated, and is preferably concentrated to an extent such that the cobalt level is at or near saturation. The aqueous concentrate advantageously contains the maximum possible amount of cobalt in solution for the particular operating temperature and for the particular cobalt salt employed. If, as is preferred, formic acid has been employed in the demetalling step, the aqueous phase is preferably concentrated to give a cobalt concentration of from 0.5 to 2 wt %, based on the total concentrate, expressed as elementary cobalt, before it is fed to the preformer. Advantageously, the concentration of free formic acid in the concentrate is from 2 to 8 wt % based on the total concentrate.

To ensure as far as possible the absence of volatile cobalt compounds from the aqueous phase sent for concentration, especially when this is effected by evaporation as is preferred, the aqueous phase is contacted with oxygen, conveniently in the form of air. If demetalling is carried out in the presence of air, this step is normally sufficient to oxidize the volatile cobalt compounds to non-volatile cobalt species. If, however, airless demetalling is employed, then a separate oxidation step, for example injecting air into the aqueous phase, may desirably be employed before concentration.

The concentrate may be produced by flashing the aqueous phase in an evaporator or by using any other convenient means, for example, a membrane, to yield the concentrate, for example, a cobalt formate/formic acid aqueous solution, and an aqueous effluent which is substantially free from cobalt provided all volatile cobalt compounds have previously been oxidized. This aqueous effluent may, as indicated above, accordingly be recycled for use in removing any traces of cobalt from the oxo product after the demetalling step, for example in a wash tower as described in U.S. Patent No. 4625067. Preferably, the water stream fed to the wash tower is exclusively aqueous effluent from the evaporator. If desired, fresh water make-up, for example, in an amount of 1 to 3% by volume, based on the oxo product, may be added to the aqueous effluent from the evaporator that is fed to the wash tower. In that manner, water from the evaporator may be retained within a substantially closed cycle, it being possible substantially to avoid the need to purge water from the evaporator. If it is necessary, for example, in order to remove undesired impurities, some aqueous effluent from the evaporator may be purged from the system.

As already mentioned it is preferred to use as the aqueous phase contacted with the crude oxo product an aqueous formic acid solution, although other acids, for example acetic acid, may be used. For ease of explanation, it is assumed in the following discussion of the preforming step that formic acid is used.

The cobalt values contained in the aqueous phase will be primarily in the form of cobalt formate, which, in the preformer, in the presence of syn gas is converted to $Co[Co(CO)_4]_2$ in accordance with the following reaction:

$$3Co(COOH)_2 + 3H_2 + 8CO \rightarrow Co[Co(CO)_4]_2 + 6HCOOH$$

Hydrocobalt carbonyl is liberated from the $Co[Co(CO)_4]_2$ by the following reaction, formation of the hydrocobalt carbonyl being enhanced by the presence of free formic acid, which is produced as a by-product in the hydroformylation process as well as in the conversion of cobalt formate to $Co[Co(CO)_4]_2$, and will normally be present in the aqueous phase:

$$Co[Co(CO)_4]_2 + 2HCOOH \rightarrow 2HCo(CO)_4 + Co(COOH)_2$$

Hydrocobalt carbonyl is relatively insoluble in water (maximum solubility at 20°C 3000 ppm, expressed as elemental cobalt). It is believed that as the concentration of the hydrocobalt carbonyl in the aqueous phase approaches a concentration corresponding to the said maximum solubility, the carbonyl separates from the aqueous phase and is extracted into the organic liquid phase. Some of the hydrocobalt carbonyl becomes entrained in the syn gas. Further hydrocobalt carbonyl may be transformed into dicobalt octacarbonyl, which is extracted into the organic liquid phase.

The organic solvent may be any organic solvent that is substantially immiscible with the aqueous phase with which it comes into contact. The solvent may, for example, be an olefin, decobalted crude oxo product (that is, the product obtained after treatment of crude oxo product to remove catalytic residues but before hydrogenation or distillation of the organic material), hydrogenated oxo product, an oxo alcohol or alcohols, or a light or heavy oxo fraction. The solvent should generally be one that is liquid under the conditions prevailing in the preformer and the stripper.

Lower molecular weight, especially $C_6$ to $C_{13}$, alcohols that are substantially immiscible with the aqueous phase under the reaction conditions are especially suitable as organic solvents. The preferred alcohols are saturated aliphatic monohydric alcohols, for example a hexanol, heptanol or octanol. Conveniently the alcohol used is an oxo process alcohol, and oxo process heptanol is especially preferred.

As indicated above, however, it is a feature of the invention that the catalyst regeneration is carried out in large part independently of the oxo process itself, and that the organic solvent and hence the liquid organic phase containing cobalt values may if desired be, and preferably are, maintained out of contact with both the olefin feed to the oxonation reactor and the oxo product. Accordingly, the organic solvent may be chosen solely on the basis of its suitability for use in facilitating recovery of the cobalt from the aqueous phase since considerations of compatibility of the solvent with the olefin feed, as in U.S. Patent No. 3 725 534, or the oxo product, as in U.S. Patent No. 4 625 067, do not apply.

This separation, resulting in the catalyst recovery being performed in "outboard" units, leads to further very important advantages. The first of these is that a single catalyst recovery system, employing, for example, a common evaporator, preformer, and stripper, may be used for two or more oxonation trains operating on different starting materials and/or producing different oxo products. This results in lower investment costs.

Secondly, the removal of the catalyst residues from the crude oxo product does not involve a stripping step. Accordingly, the associated problem of build up of unreactive species in the olefin feed does not arise when lighter grades of olefins are being oxonated, the stripping stage of the method according to the invention being carried out in the absence of both feedstock to, and the product of, the oxonation reaction.

The ratio of the solvent chosen to form the organic liquid phase to aqueous phase in the preforming step is advantageously from 0.2:1 to 5:1 by volume and may, for example, be 1:1 by volume. The amount of syn gas is preferably so selected that both hydrogen and carbon monoxide are present in a stoichiometric excess of at least 20% over the total cobalt in the preformer feed. The molar ratio of hydrogen to carbon monoxide may be from 0.5:1 to 2:1.

If catalytic preforming is employed, the aqueous phase is advantageously combined with the organic solvent before entering the preformer, and the combined aqueous and organic liquid phases obtained are passed to a two-liquid-phase plug flow reactor having one or more fixed beds of Pd/C, to which syn gas is also fed. The reactor comprises means, e.g., a distribution plate, for effecting mixing of the two liquid phases and the gas phase so that the two liquid phases are well dispersed and the interface between the liquid phases and the gas phase is sufficiently large that the mass transfer of material between the liquid phases and the gas phase is not limited by the size of the said interface. The reactor also comprises means for effecting good catalyst wetting by both the liquid phases and the gaseous phases; this may be achieved by employing a reactor with a high length:diameter ratio so as to achieve, advantageously, a total liquid throughput, i.e., combined aqueous and organic phases, of at least 4.1 kg/m$^2$/s. The temperature in the reactor is preferably from 50 to 175°C, more preferably from 120 to 140°C. The pressure in the reactor may be from 10 to 30 MPa, and is preferably from 25 to 30 MPa.

If a catalyst, e.g., 2% Pd on carbon is employed, a residence time of 0.2 to 1.0, preferably 0.4 to 0.8, hr is advantageously employed.

Alternatively, and preferably, the reaction is carried out in the absence of a catalyst in a tubular reactor with a very high length: diameter ratio with turbulence, as mentioned above. The reaction is advantageously assisted to commence by incorporating an initiator in one or both of the liquid phases, either $HCo[CO]_4$ or $CO_2(CO)_8$ in the organic phase or $Co[Co(CO)_4]_2$ in the aqueous phase, at a concentration of up to 3000 wppm, advantageously from 1000 to 3000 wppm, measured as cobalt.

In the tubular reactor without catalyst, reaction pressures as given above with reference to catalysed preforming are appropriate. Advantageously, the reaction is carried out at a temperature within the range of 50 to 200°, and preferably from 120 to 175°C. In a preferred method of preforming, a first stage is carried out at a higher temperature, for example 150 to 175°C, and a second stage is carried out at a lower temperature, for example from 20 to 120°C, preferably from 50 to 110°C. By this reduction in temperature, while maintaining a high pressure, more cobalt carbonyl is transferred to the organic phase, allowing further reaction to take place in the aqueous phase.

If a tubular reactor, i.e., one providing a high 1:d ratio is employed, residence time may be considerably shorter than if catalytic preforming is carried out. If this procedure is adopted, the total residence time is advantageously from 2 to 10 minutes, including both first and second stages if both are employed. The total residence time in the first, or the sole, high temperature stage is advantageously from 90 seconds to 5 minutes, preferably from 2 to 5 minutes. If the second low temperature stage is employed the residence time is advantageously from 30 seconds to 5 minutes.

Because the preferred preforming cycle described above allows essentially all the water to be in a closed loop, the waste water from the evaporator being recycled, preferably via a wash tower, to the stripping step, the need to dispose of waste water may be substantially avoided. Thus, the method of the invention enables environmentally clean cobalt recovery to be attained.

Further, because the invention provides an internal closed loop for any cobalt formate in the aqueous phase that remains unreacted in the preformer, it is not important for complete preforming conversion to be obtained. Cobalt values that have not been preformed (that is, converted to cobalt in an active form) are retained in the aqueous phase and recycled.

Also, the process may be operated without the addition of chemical reactants, other than the reactants for use in the oxo process, because formic acid, which promotes the conversion of $Co[Co(CO)_4]_2$ to hydrocobalt carbonyl, is produced in the oxo reaction. Thus, after an initial introduction of formic acid, it is possible to make the system substantially closed with regard to formic acid.

Using a tubular reactor as preformer, an overall cobalt recovery of about 50 % based on the cobalt entering the preformer, may be achieved, with approximately 70 %, or up to 85 % including an equilibration step, of cobalt values being converted in the preformer to volatile cobalt carbonyls and about 70 % of that preformed cobalt being taken up by the stripping gas in the stripping step.

Any cobalt loss may be made up by introducing cobalt, for example, in the form of cobalt soap, into the preformer with the organic phase or in the form of an organic cobalt salt, for example, cobalt formate, in aqueous solution.

Two methods of cobalt recovery in accordance with the invention will now be described in greater detail by way of example only with reference to the accompanying drawings of which:

Figure 1 is a flow diagram of a hydroformylation reaction system in which demetalling of the product is carried out in the presence of air, and
Figure 2 is a flow diagram of a hydroformylation reaction system in which the demetalling is carried out in the absence of air.

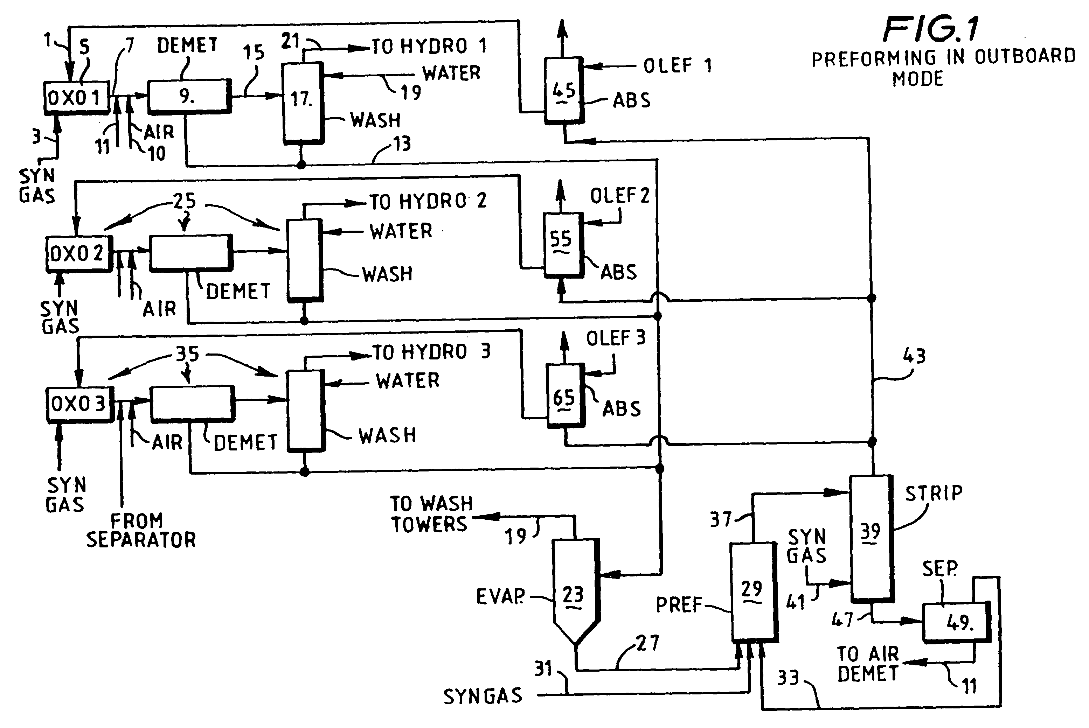

Referring to Figure 1, a first feed olefin is delivered via a line 1 to a first oxo reactor 5, to which is also delivered syn gas via a line 3. Hydroformylation takes place within the reactor, and a crude product containing aldehydes, alcohols, cobalt catalyst compounds and by-products is carried via a line 7 to a demetallizer unit 9, air being introduced through a line 10 and an aqueous washing phase through a line 11. The aqueous washing phase is a dilute formic acid solution. In the unit 9 the aqueous and organic phases are separated, the aqueous phase, which contains the major proportion of the cobalt, being fed by a line 13 to an evaporator 23, while the organic phase is led by a line 15 to a wash tower 17. In the tower 17, the organic phase is washed with cobalt-free water delivered by a line 19, and the now substantially cobalt-free organic phase is fed by a line 21 to a hydrogenation unit, not shown. The reactor 5, the unit 9, and the tower 17 and associated lines form a first oxonation train.

The cobalt-containing wash water from the tower 17 is fed by the line 13 to the evaporator 23 together with the aqueous phase from the unit 9. Also shown in Figure 1, and indicated generally by the reference numerals 25 and 35, are second and third oxonation trains, in which generally similar procedures are carried out as in the above-described first oxonation train but, if desired, on different olefin feeds or under different operating conditions to yield different products.

The cobalt-containing aqueous phase is concentrated in the evaporator 23, the distillate water being fed by the line 19 to the wash towers of the three oxonation trains. The cobalt concentrate is fed by a line 27 to a preformer unit 29, into which is also fed syn gas through a line 31 and an organic solvent through a line 33, the organic solvent being substantially immiscible with water and a good solvent for dicobalt octacarbonyl and hydrocobalt carbonyl. The liquids being fed to the preformer unit 29 accordingly are an aqueous phase containing formic acid and cobalt formate, and an organic phase containing, for example, a $C_6$ to $C_{13}$ alcohol also containing formic acid and formate esters.

In the preformer, which may be catalyst-free or may contain a catalyst, e.g., palladium on charcoal, the cobalt is converted to dicobalt octacarbonyl and hydrocobalt carbonyl. The reaction product from the preformer 29 is fed by a line 37 to a stripper 39, to which is also fed syn gas by a line 41, the syn gas entraining the volatile cobalt carbonyls being taken off overhead and led by a line 43 to each of three absorber units 45, 55 and 65, where it meets the feed olefin to the respective oxonation train. The bottoms product from the stripper 39 is fed by a line 47 to a separator 49. The aqueous phase, which has now had a substantial proportion of cobalt removed and comprises a dilute formic acid solution, is fed from the separator 49 by the line 11 to the demetalling unit 9 or to the evaporator 23. The organic phase, which comprises the $C_6$ to $C_{13}$ alcohol and formate esters, is fed by the line 33 to the preformer 41.

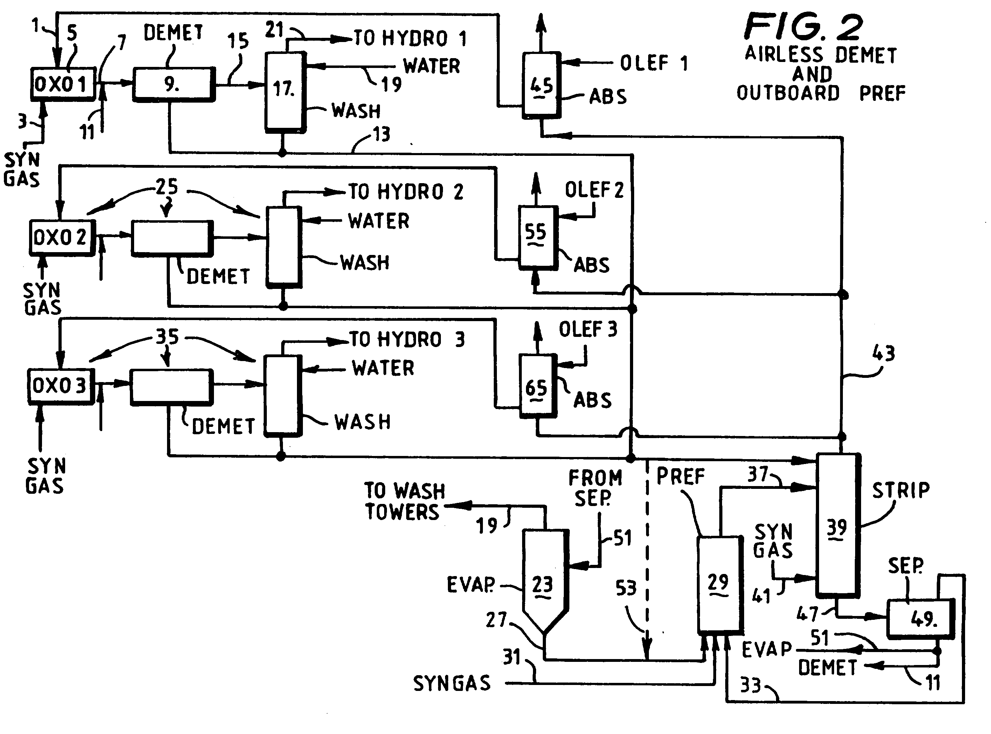

Referring now to Fig. 2, in which units and lines identical to those in Fig. 1 are given the same reference numerals, the oxonation trains are similar to those of Fig. 1 except that the air line to the demetallizer unit 9 is omitted. The line 13 carrying the aqueous phase containing cobalt values from the unit 9 and the wash tower 17 leads to the stripper 39 rather than to the evaporator 23, to which, as in Fig. 1, the line 37 carrying the preformer product and syn gas line 41 also lead. Optionally, but advantageously, a portion of the cobalt-containing aqueous phase from line 13 may be fed through a line 53 to the preformer. In this way, the cobalt content of the stream that is in the form of hydrocobalt carbonyl assists in initiating preforming, which is especially helpful if a non-catalytic preforming step is used.

The aqueous phase from the separator is split, part of the formic acid solution being led by line 11 to the demetallizer unit 9 and part by line 51 to the evaporator 23. As before, the cobalt-free product from the evaporator is led by line 19 to the wash towers. If desired, an oxidation unit (not shown) may be provided to oxidize any cobalt volatiles remaining in the aqueous phase fed to the evaporator through line 51.

In this procedure, in which no air is employed in the demetalling step, the oxo product is demetalled using only water, a low molecular weight carboxylic acid, and/or a cobalt salt solution of such an acid, binding most of the cobalt catalyst as hydrocobalt carbonyl, which, as indicated above, assists in the preforming step, especially if a non-catalytic reactor is used.

Cobalt catalyst is regenerated by the procedure according to the invention, and all aqueous phase may be recycled, avoiding the need for effluent disposal.

Example 1

In this Example, catalyst-free batch preforming of an aqueous cobalt solution was effected in the presence of a $C_7$ oxo alcohol. The cobalt was primarily present as formate, but 0.3% by weight hydrocobalt carbonyl was present as initiator.

The reactor was a 4 litre autoclave equipped with a thermostatically controlled heater, pressure regulator, and gas and liquid phase injector lines. Stirring was carried out by a 6-blade 3000 r.p.m. (max) turbine impeller in conjunction with removable baffles in the vessel.

The alcohol, 820g, containing 3000 ppm cobalt as hydrocobalt carbonyl, was introduced into the reactor, the reactor purged with syn gas (mole ratio $H_2$:CO of 1:2) with stirring at 1800 r.p.m. and the pressure raised to 30MPa, and the temperature to 175°C. 1027 g of aqueous cobalt formate (pH 3.2; 1.067% $Co^{2+}$ as formate) were introduced, and the progress of the reaction monitored by taking samples from the alcohol and water phases and analysing them for cobalt. The results are given in Table 1 below.

TABLE 1

| | Starting materials | Equilibrium after Reaction Time, mins | | | |
| --- | --- | --- | --- | --- | --- |
| | | 0 | 4 | 8 | 30 |
| Organic, g | 820 | 943 | 943 | 943 | 943 |
| Water*, g | 1027 | 904 | 862 | 779 | 737 |
| Wt% Co in organic | 0.3068 | 0.2668 | | | 0.1622 |
| GMOL Co (org) | 0.0378 | 0.0378 | | | 0.0230 |
| GMOL $Co_2(CO)_8$ | | | | | 0.0013 |
| GMOL $HCo(CO)_4$ | | | | | 0.0216 |
| Wt% Co in water | 1.0675 | 1.2127 | 1.2885 | 1.4096 | 1.3476 |
| Wt% $Co^{2+}$ | | 1.2127 | 0.3850 | 0.3534 | 0.3408 |
| GMOL $Co^{2+}$ | | 0.2056 | 0.0653 | 0.0599 | 0.0578 |
| GMOL $Co^{-1}$ | | 0.0000 | 0.1531 | 0.1790 | 0.1706 |
| Cobalt balance, % | | 100.00 | | | 101.77 |
| $Co^{2+}$ Conv, % | | 0.00 | 68.11 | 72.74 | 74.76 |

* Reduction largely due to sampling loss

The results indicate that, under the conditions shown, equilibrium conversion (about 70%) of $Co^{2+}$ to $Co^{-1}$ is reached after about 6 minutes.

Example 2

The procedure of Example 1 is repeated, except that the reactor temperature was 150°C, and the initial wt% cobalt in the water was 1.2682. The results are given in Table 2.

EP 0 642 488 B1

TABLE 2

| | Starting materials | Equilibrium after Reaction Time, mins | | | |
|---|---|---|---|---|---|
| | | 0 | 4 | 8 | 30 |
| Organic, g | 840 | 932.4 | 932.4 | 932.4 | 932.4 |
| Water, g | 1050 | 957.6 | 917.6 | 839.6 | 759.6 |
| Wt% Co in organic | 0.2967 | 0.2673 | | | 0.3279 |
| GMOL Co (org) | | 0.378 | | | 0.0464 |
| GMOL $Co_2(CO)_8$ | | | | | 0.0076 |
| GMOL $HCo(CO)_4$ | | | | | 0.0388 |
| Wt% Co in water | 1.2682 | 1.3906 | 1.2887 | 1.0079 | 1.2194 |
| Wt% $Co^{2+}$ | | 1.3906 | 1.0242 | 0.2896 | 0.2481 |
| GMOL $Co^{2+}$ | | 0.2357 | 0.1736 | 0.0491 | 0.0421 |
| GMOL $Co^{-1}$ | | 0.0000 | 0.0448 | 0.1217 | 0.1646 |
| Cobalt balance, % | | 100.00 | | | 92.2 |
| $Co^{2+}$ Conv, % | | 0.00 | 26.84 | 80.10 | 84.30 |

The results show that at 150°C it takes longer for equilibrium to be reached but that the equilibrium conversion ratio is higher.

Example 3

The procedure of Example 1 is repeated, except that the concentration of hydrocobalt carbonyl in the alcohol was 1000 ppm, and the initial weight % cobalt in the water was 1.1018. The % conversion is given in Table 3 below. A longer period than in Example 1 is required for equilibrium conversion to be reached.

Table 3

| | Time, Minutes | | | | | | | |
|---|---|---|---|---|---|---|---|---|
| | 0 | 2 | 4 | 6 | 8 | 10 | 15 | 30 |
| $Co^{2+}$ Conversion % | 0 | 8.73 | 22.26 | 74.18 | 77.26 | 78.19 | 79.50 | 80.67 |

Example 4

In this Example, no hydrocobalt carbonyl was initially present in the alcohol, and initially, the reactor temperature was as in Example 1. After 20 minutes, however, it was reduced to 120°C. Table 4 shows that in the first 7 minutes, when no initiator was present at start-up, reaction was relatively slow. Between 7 and 10 minutes, however, the reaction was much more rapid, being catalysed by the initiator produced by the reaction. The equilibrium shift produced by the reduction in temperature after 20 minutes is also shown.

TABLE 4

| | Starting materials | Equilibrium after Reaction Time, mins | | | | | |
|---|---|---|---|---|---|---|---|
| | | 0 | 7 | 10 | 15 | 20 | 45 |
| Organic, g | 847 | 974.05 | 956.55 | 939.05 | 921.55 | 904.05 | 832.31 |
| Water*, g | 1034 | 906.95 | 862.95 | 816.95 | 771.95 | 726.95 | 781.19 |
| Wt% Co in organic | 0 | 0.0000 | 0.0443 | 0.2485 | 0.4379 | 0.1419 | 0.4240 |
| GMOL Co (org) | 0 | 0.0000 | | | | | 0.0600 |
| GMOL $Co_2(CO)_8$ | | | | | | | 0.0189 |
| GMOL $HCo(CO)_4$ | | | | | | | 0.0411 |
| Wt% Co in water | 1.0329 | 1.1776 | 1.1679 | 1.0466 | 0.9720 | 1.0934 | 0.6429 |
| Wt% $Co^{2+}$ | | 1.1776 | 0.9985 | 0.5784 | 0.2689 | 0.2538 | 0.0968 |
| GMOL $Co^{2+}$ | | 0.1996 | 0.1692 | 0.0980 | 0.0456 | 0.0430 | 0.0164 |
| GMOL $Co^{-1}$ | | 0.0000 | 0.0287 | 0.0794 | 0.1192 | 0.1423 | 0.0926 |
| Cobalt balance, % | | 100.00 | 104.13 | 114.18 | 127.84 | 108.45 | 107.50 |
| $Co^{2+}$ Conv, % | | 0.00 | 15.36 | 51.39 | 78.06 | 80.25 | 91.80 |

The batch procedures of Examples 1 to 4 show that with the good contact between the phases obtained by high speed stirring equilibrium conditions are reached rapidly at high temperatures and in the presence of an auto-initiator in a liquid phase. However, a more favourable equilibrium exists at lower temperatures.

Example 5

In this Example, continuous preforming is illustrated. The process was carried out in a stainless steel (Type 316L) tubular reactor, of length 110 m, internal diameter 1.1 mm, and providing a free space of 1.04 dl. The reactor was maintained at 175°C through external steam heating. The inlet pressure was maintained at 30 MPa. The reactants were fed into the preformer for 3 hours under the conditions indicated in Table 5.

TABLE 5

| | |
|---|---|
| Oxo process heptanol containing 0.2537 Wt % Cobalt as $HCo[CO]_4$ | 0.58 litre/h |
| Syn gas ($H_2$:Co 1:2) | 1.6 litres/h |
| Cobalt water (containing 0.9476 Wt % cobalt as formate) | 0.519 litre/h |
| Residence Time | 2.34 mins |

Under these conditions, an outlet pressure of 28.5 MPa was obtained. The pressure drop of 1.5 MPa ensures intensive contact between the phases.

The reaction mixture leaving the preformer was fed into a stripper operated under the conditions shown, and producing a reaction product as shown, in Table 6.

TABLE 6

| | | |
|---|---|---|
| Temperature | | 100°C |
| Pressure | | 80 KPa |
| Preformer Organic Phase | | 0.557 litre/h |
| Preformer Aqueous Phase | | 0.527 litre/h |
| Added water (5% acid) | | 0.25 litre/h |
| Syn Gas | | 148 litre/h |
| Product, Cobalt content, | Wt% | Gm |
| Water at bottom, $Co^{2+}$ | 0.418 | 8.55 |
| Water at bottom, $Co^{-1}$ | 0 | 0 |
| Organic at bottom | 0.0027 | 0.03 |
| Overhead recovery | | 10.06 |
| % per pass conversion | | 41.99 |

Example 6

The percentage conversion per pass in Example 5, about 42%, may be improved by taking advantage of the equilibrium shift that takes place on reducing the temperature, as in Example 4. In the present Example, a reactor maintained at 175°C is provided by three stirred autoclaves in series, the autoclaves being as described in Example 1. By providing reaction conditions of high stirring rate and long residence time, equilibrium conditions were assured (and thereby a basis of comparison for Example 5 is provided, giving confirmation that reaction equilibrium at the prevailing temperature and pressure had been reached in the tubular reactor in that Example).

The reaction mixture from the final autoclave was passed to the tubular reactor which was maintained at 107°C. The results are shown in Table 7.

## TABLE 7

### Preforming Zone 1 (Three stirred Autoclaves)

| | |
|---|---|
| Pressure | 30 MPa |
| Syn Gas ratio $H_2$:CO | 1:2 |
| Stirring rate | 1500 RPM |
| Organic feed rate (iso-heptanol) | 0.461 l/h |
| Cobalt content | 0.2404 wt% |
| Aqueous feed rate | 0.425 l/h |
| Cobalt content | 1.0744 wt% |
| Residence Time | 163 mins |
| Output - cobalt content, aqueous phase | 0.3475 wt% |
| Conversion rate | 70.8% |

### Preforming Zone 2 (Tubular Reactor)

| | |
|---|---|
| Inlet Pressure | 30 MPa |
| Outlet Pressure | 28 MPa |
| Residence Time | 5 mins |
| Output - cobalt content, aqueous phase | 0.2382 wt% |
| Conversion rate | 78.2% |

### Stripper Zone

| | |
|---|---|
| Temperature | 100°C |
| Pressure | 80 KPa |
| Feed rate - organic phase | 0.443 g/l |

| | |
|---|---|
| Feed rate - preformer water | 0.425 g/l |
| Make up water | 0.174 g/l |
| Gas | 134 g/l |

**Stripper Product, cobalt content**

| | |
|---|---|
| Water at bottom, $Co^{2+}$ | 0.4483 wt% |
| Water at bottom, $Co^{-1}$ | 0 wt% |
| Organic at bottom | 0.0057 wt% |
| Overhead recovery | 10.38 g |
| % per pass conversion | 48.4% |

Example 7

The procedure of Example 5 was repeated, using identical starting materials in similar proportions, but with a residence time in the preformer of 4.8 minutes, with no cobalt in the organic phase, but with 1054 ppm $Co^{-1}$ as initiator in the aqueous phase, other conditions remaining substantially the same as in Example 5. The per cent per pass conversion was 50.1, based on the cobalt formate in the water fed to the preformer, indicating that an initiator in the aqueous phase is also effective.

Example 8

The procedure of Example 7 was repeated, using as the organic phase iso-heptanol containing 14 % of formates and 1.14 % formic acid. 773 ppm $Co^{-1}$ were present in the aqueous phase. The per cent per pass conversion fell to 41.8. This example illustrates that a 14% level of formate in the iso-heptanol causes a fall in conversion rate.

Example 9

The procedure of Example 8 was repeated using the same cobalt-free organic phase but the aqueous phase contained cobtalt formate and no initiator. The per pass conversion fell to 39.6%, indicating that even without initiator present in either liquid phase conversion takes place, albeit at a lower rate.

Example 10

The procedure of Example 7 was repeated, but using iso-decanol as the organic phase. The per cent per pass conversion was 36.0, indicating the lower effectiveness of the higher molecular weight alcohol.

**Claims**

1. A method for recovering cobalt values from a liquid organic phase containing them, the method comprising contacting the liquid organic phase with an aqueous phase of pH at most 7 that is substantially immiscible with the liquid organic phase and capable of extracting cobalt values from the organic phase, and separating the resulting cobalt-containing aqueous phase from the cobalt-depleted organic phase,

   contacting the separated cobalt-containing aqueous phase with syn gas and an organic solvent for dicobalt octacarbonyl and hydrocobalt carbonyl, the organic solvent being substantially immiscible with the aqueous phase, and recovering from the aqueous phase and the organic phase so obtained volatile cobalt compounds, separating the resultant aqueous and organic phases,

returning the organic phase to form the said organic solvent and
returning the aqueous phase to form the said aqueous phase of pH at most 7.

2. A method as claimed in claim 1, wherein the cobalt-containing aqueous phase is concentrated to form a concentrated cobalt-containing aqueous phase and a substantially cobalt-free aqueous phase before the concentrated cobalt-containing aqueous phase is contacted with syn gas and organic solvent.

3. A method as claimed in claim 2, wherein the cobalt-containing aqueous phase is concentrated to bring the cobalt concentration to a level at or near saturation at ambient temperature.

4. A method as claimed in claim 2 or claim 3, wherein the aqueous phase is contacted with oxygen before concentration.

5. A method as claimed in any one of claims 1 to 4, wherein the liquid organic phase is the crude product of a cobalt-catalysed oxo reaction.

6. A method as claimed in claim 5, wherein contact between the crude product and the aqueous phase of pH at most 7 takes place in the presence of oxygen.

7. A method as claimed in claim 5, wherein contact between the crude product and the aqueous phase of pH at most 7 takes place in the substantial absence of oxygen.

8. A method as claimed in claim 7, wherein the separated cobalt-containing aqueous phase is fed to a stripper and contacted therein with a stripping gas and the product of reaction of a cobalt-containing aqueous phase, syn gas, and the organic solvent for dicobalt carbonyl and hydrocobalt carbonyl; the thus-treated aqueous phase is recovered as bottoms from the stripper and separated from the liquid organic phase before being contacted with syn gas and the organic solvent.

9. A method as claimed in any one of claims 1 to 8, wherein the aqueous phase of pH at most 7 is a dilute formic acid solution.

10. A method as claimed in any one of claims 1 to 9, wherein the organic solvent is a hydrogenated oxo product.

11. A method as claimed in any one of claims 1 to 10, wherein the organic solvent is an alcohol having from 6 to 13 carbon atoms.

12. A method as claimed in claim 11, wherein the organic solvent is a heptanol.

13. A method as claimed in any one of claims 1 to 12, wherein the contact between the cobalt-containing aqueous phase, syn gas, and organic solvent takes place in the presence of a catalyst.

14. A method as claimed in claim 13, wherein the catalyst is on a fixed bed.

15. A method as claimed in claim 13 or claim 14, wherein the catalyst comprises palladium.

16. A method as claimed in any one of claims 1 to 12, wherein the contact between the cobalt-containing aqueous phase, syn gas, and organic solvent takes place in the absence of a catalyst in conditions of turbulent flow.

17. A method as claimed in claim 16, wherein the contact takes place in a tubular reactor having a high length:diameter ratio.

18. A method as claimed in claim 17, wherein the ratio is at least 100.

19. A method as claimed in claim 18, wherein the ratio is within the range of from 1,000 to 100,000.

20. A method as claimed in any one of claims 1 to 19, wherein the contact of the separated cobalt-containing aqueous phase with syn gas and the organic solvent is carried out in two stages, the first at a higher temperature and the second at a lower temperature.

21. A method as claimed in claim 20, wherein the higher temperature is within the range of from 150 to 175°C, and the lower temperature is within the range of from 20 to 120°C.

22. A method as claimed in any one of claims 1 to 21, wherein an initiator is present for the reaction between the cobalt-containing aqueous phase, syn gas, and organic solvent.

23. A method as claimed in claim 22, wherein the initiator is hydrocobalt carbonyl or dicobalt octacarbonyl in the organic solvent.

24. A method as claimed in claim 22 or claim 23, wherein the initiator is cobalt carbonyl in the aqueous phase.

25. A method as claimed in any one of claims 1 to 21, wherein no initiator is present for the reaction between the cobalt-containing aqueous phase, syn gas, and organic solvent.

26. A method as claimed in any one of claims 1 to 25, wherein volatile cobalt compounds are recovered by stripping.

27. A method as claimed in claim 26, wherein stripping is carried out using syn gas.

28. A method as claimed in claim 27 wherein the cobalt-containing syn gas is contacted with olefin feed to an oxo reactor, cobalt values in the gas dissolving in or become entrained in the olefin feed.

29. A method as claimed in any one of claims 1 to 28, which is carried out on a liquid organic phase that is the product of a plurality of cobalt-catalysed oxo reactions.

**Patentansprüche**

1. Verfahren zur Rückgewinnung von Kobaltbestandteilen aus einer flüssigen organischen Phase, die diese enthält, bei dem die flüssige organische Phase mit einer wäßrigen Phase mit einem pH-Wert von höchstens 7 kontaktiert wird, die mit der flüssigen organischen Phase im wesentlichen unmischbar ist und in der Lage ist, Kobaltbestandteile aus der organischen Phase zu extrahieren, und die resultierende kobalthaltige wäßrige Phase von der an Kobalt verarmten organischen Phase abgetrennt wird,

   die abgetrennte kobalthaltige wäßrige Phase mit Syngas und einem organischen Lösungsmittel für Dikobaltoctacarbonyl und Hydrokobaltcarbonyl kontaktiert wird, wobei das organische Lösungsmittel im wesentlichen unmischbar mit der wäßrigen Phase ist, und aus der wäßrigen Phase und der so erhaltenen organischen Phase flüchtige Kobaltverbindungen gewonnen werden,
   die resultierenden wäßrigen und organischen Phasen getrennt werden,
   die organische Phase zurückgeführt wird, um das organische Lösungsmittel zu bilden, und
   die wäßrige Phase zurückgeführt wird, um die wäßrige Phase mit einem pH-Wert von höchstens 7 zu bilden.

2. Verfahren nach Anspruch 1, bei dem die kobalthaltige wäßrige Phase aufkonzentriert wird, um eine konzentrierte kobalthaltige wäßrige Phase und eine im wesentlichen kobaltfreie wäßrige Phase zu bilden, bevor die konzentrierte kobalthaltige wäßrige Phase mit Syngas und organischem Lösungsmittel kontaktiert wird.

3. Verfahren nach Anspruch 2, bei dem die kobalthaltige wäßrige Phase aufkonzentriert wird, um die Kobaltkonzentration auf ein Niveau der Sättigung oder nahezu der Sättigung bei Umgebungstemperatur zu bringen.

4. Verfahren nach Anspruch 2 oder Anspruch 3, bei dem die wäßrige Phase vor der Aufkonzentrierung mit Sauerstoff kontaktiert wird.

5. Verfahren nach einem der Ansprüche 1 bis 4, bei dem die flüssige organische Phase das Rohprodukt einer kobaltkatalysierten Oxoreaktion ist. .

6. Verfahren nach Anspruch 5, bei dem der Kontakt zwischen dem Rohprodukt und der wäßrigen Phase mit einem pH-Wert von höchstens 7 in Gegenwart von Sauerstoff stattfindet.

7. Verfahren nach Anspruch 5, bei dem der Kontakt zwischen dem Rohprodukt und der wäßrigen Phase mit einem pH-Wert von höchstens 7 in wesentlicher Abwesenheit von Sauerstoff stattfindet.

8. Verfahren nach Anspruch 7, bei dem die abgetrennte kobalthaltige wäßrige Phase in einen Stripper eingespeist wird und dort mit einem Strippgas und dem Produkt der Umsetzung einer kobalthaltigen wäßrigen Phase, Syngas und dem organischen Lösungsmittel für Dikobaltcarbonyl und Hydrokobaltcarbonyl kontaktiert wird, wobei die so behandelte wäßrige Phase als Bodenprodukt aus dem Stripper gewonnen wird und von der flüssigen organischen Phase abgetrennt wird, bevor sie mit dem Syngas und dem organischen Lösungsmittel kontaktiert wird.

9. Verfahren nach einem der Ansprüche 1 bis 8, bei dem die wäßrige Phase mit einem pH-Wert von höchstens 7 eine verdünnte Ameisensäurelösung ist.

10. Verfahren nach einem der Ansprüche 1 bis 9, bei dem das organische Lösungsmittel ein hydriertes Oxoprodukt ist.

11. Verfahren nach einem der Ansprüche 1 bis 10, bei dem das organische Lösungsmittel ein Alkohol mit 6 bis 13 Kohlenstoffatomen ist.

12. Verfahren nach Anspruch 11, bei dem das organische Lösungsmittel ein Heptanol ist.

13. Verfahren nach einem der Ansprüche 1 bis 12, bei dem der Kontakt zwischen der kobalthaltigen wäßrigen Phase, Syngas und dem organischen Lösungsmittel in Gegenwart eines Katalysators stattfindet.

14. Verfahren nach Anspruch 13, bei dem der Katalysator sich auf einem Festbett befindet.

15. Verfahren nach Anspruch 13 oder Anspruch 14, bei dem der Katalysator Palladium umfaßt.

16. Verfahren nach einem der Ansprüche 1 bis 12, bei dem der Kontakt zwischen der kobalthaltigen wäßrigen Phase, Syngas und organischem Lösungsmittel in Abwesenheit eines Katalysators unter Bedingungen turbulenter Strömung stattfindet.

17. Verfahren nach Anspruch 16, bei dem der Kontakt in einem Rohrreaktor mit einem hohen Verhältnis von Länge zu Durchmesser stattfindet.

18. Verfahren nach Anspruch 17, bei dem das Verhältnis mindestens 100 beträgt.

19. Verfahren nach Anspruch 18, bei dem das Verhältnis im Bereich von 1 000 bis 100 000 liegt.

20. Verfahren nach einem der Ansprüche 1 bis 19, bei dem der Kontakt der abgetrennten kobalthaltigen wäßrigen Phase mit Syngas und dem organischen Lösungsmittel in zwei Stufen durchgeführt wird, wobei die erste bei einer höheren Temperatur und die zweite bei einer niedrigeren Temperatur durchgeführt wird.

21. Verfahren nach Anspruch 20, bei dem die höhere Temperatur im Bereich von 150 bis 175°C liegt und die niedrigere Temperatur im Bereich von 20 bis 120°C liegt.

22. Verfahren nach einem der Ansprüche 1 bis 21, bei dem für die Reaktion zwischen der kobalthaltigen wäßrigen Phase, Syngas und organischem Lösungsmittel ein Initiator vorhanden ist.

23. Verfahren nach Anspruch 22, bei dem der Initiator Hydrokobaltcarbonyl oder Dikobaltoctacarbonyl in dem organischen Lösungsmittel ist.

24. Verfahren nach Anspruch 22 oder Anspruch 23, bei dem der Initiator Kobaltcarbonyl in der wäßrigen Phase ist.

25. Verfahren nach einem der Ansprüche 1 bis 21, bei dem für die Reaktion zwischen der kobalthaltigen wäßrigen Phase, Syngas und organischem Lösungsmittel kein Initiator vorhanden ist.

26. Verfahren nach einem der Ansprüche 1 bis 25, bei dem flüchtige Kobaltverbindungen durch Strippen gewonnen werden.

27. Verfahren nach Anspruch 26, bei dem das Strippen unter Verwendung von Syngas durchgeführt wird.

**28.** Verfahren nach Anspruch 27, bei dem das kobalthaltige Syngas mit Olefineinsatzmaterial für einen Oxoreaktor kontaktiert wird, wobei sich Kobaltbestandteile in dem Gas auflösen oder in dem Olefineinsatzmaterial mitgerissen werden.

**29.** Verfahren nach einem der Ansprüche 1 bis 28, das mit einer flüssigen organischen Phase durchgeführt wird, die das Produkt mehrerer kobaltkatalysierter Oxoreaktionen ist.

**Revendications**

**1.** Procédé pour séparer des composés de cobalt de valeur d'une phase organique liquide les contenant, procédé qui comprend les étapes consistant à mettre en contact la phase organique liquide avec une phase aqueuse à un pH d'au plus 7 qui est pratiquement non miscible à la phase organique liquide et qui est capable d'extraire les composés de cobalt de valeur de la phase organique, et à séparer la phase aqueuse contenant du cobalt résultant de la phase organique appauvrie en cobalt,

à mettre en contact la phase aqueuse séparée contenant du cobalt avec du gaz de synthèse et un solvant organique pour le dicobalt-octacarbonyle et l'hydrocobalt-carbonyle, le solvant organique étant pratiquement non miscible à la phase aqueuse, et à séparer de la phase aqueuse et de la phase organique les composés de cobalt volatils ainsi obtenus,
à séparer la phase aqueuse et la phase organique résultantes,
à renvoyer la phase organique pour former ledit solvant organique, et
à renvoyer la phase aqueuse pour former ladite phase aqueuse à un pH d'au plus 7.

**2.** Procédé suivant la revendication 1, dans lequel la phase aqueuse contenant du cobalt est concentrée pour former une phase aqueuse concentrée contenant du cobalt et une phase aqueuse pratiquement dépourvue de cobalt avant la mise en contact de la phase aqueuse concentrée contenant du cobalt avec du gaz de synthèse et un solvant organique.

**3.** Procédé suivant la revendication 2, dans lequel la phase aqueuse contenant du cobalt est concentrée pour porter la concentration de cobalt à une valeur égale à ou proche de la saturation à température ambiante.

**4.** Procédé suivant la revendication 2 ou la revendication 3, dans lequel la phase aqueuse est mise en contact avec de l'oxygène avant concentration.

**5.** Procédé suivant l'une quelconque des revendications 1 à 4, dans lequel la phase organique liquide consiste en le produit brut d'une réaction oxo catalysée par le cobalt.

**6.** Procédé suivant la revendication 5, dans lequel le contact entre le produit brut et la phase aqueuse à un pH d'au moins 7 s'effectue en présence d'oxygène.

**7.** Procédé suivant la revendication 5, dans lequel le contact entre le produit brut et la phase aqueuse à un pH d'au plus 7 s'effectue pratiquement en l'absence d'oxygène.

**8.** Procédé suivant la revendication 7, dans lequel la phase aqueuse séparée contenant du cobalt est amenée à une colonne d'extraction et est mise en contact dans cette colonne d'extraction avec un gaz d'extraction et le produit de réaction d'une phase aqueuse contenant du cobalt, de gaz de synthèse et du solvant organique pour le dicobalt-carbonyle et l'hydrocobalt-carbonyle; la phase aqueuse ainsi traitée est recueillie comme phase résiduaire de la colonne d'extraction et est séparée de la phase organique liquide avant d'être mise en contact avec le gaz de synthèse et le solvant organique.

**9.** Procédé suivant l'une quelconque des revendications 1 à 8, dans lequel la phase aqueuse à un pH d'au plus 7 consiste en une solution d'acide formique diluée.

**10.** Procédé suivant l'une quelconque des revendications 1 à 9, dans lequel le solvant organique est un produit hydrogéné de réaction oxo.

**11.** Procédé suivant l'une quelconque des revendications 1 à 10, dans lequel le solvant organique est un alcool ayant 6 à 13 atomes de carbone.

12. Procédé suivant la revendication 11, dans lequel le solvant organique est un heptanol.

13. Procédé suivant l'une quelconque des revendications 1 à 12, dans lequel le contact entre la phase aqueuse contenant du cobalt, le gaz de synthèse et le solvant organique s'effectue en présence d'un catalyseur.

14. Procédé suivant la revendication 13, dans lequel le catalyseur est présent sur un lit fixe.

15. Procédé suivant la revendication 13 ou la revendication 14, dans lequel le catalyseur comprend du palladium.

16. Procédé suivant l'une quelconque des revendications 1 à 12, dans lequel le contact entre la phase aqueuse contenant du cobalt, le gaz de synthèse et le solvant organique s'effectue en l'absence de catalyseur dans des conditions d'écoulement turbulent.

17. Procédé suivant la revendication 16, dans lequel le contact s'effectue dans un réacteur tubulaire ayant un rapport longueur : diamètre élevé.

18. Procédé suivant la revendication 17, dans lequel le rapport est égal à au moins 100.

19. Procédé suivant la revendication 18, dans lequel le rapport est compris dans l'intervalle de 1000 à 100 000.

20. Procédé suivant l'une quelconque des revendications 1 à 19, dans lequel le contact de la phase aqueuse séparée contenant du cobalt avec le gaz de synthèse et le solvant organique est effectué dans deux étages, le premier à une température plus élevée et le second à une température plus basse.

21. Procédé suivant la revendication 20, dans lequel la température plus élevée est comprise dans l'intervalle de 150 à 175°C et la température plus basse est comprise dans l'intervalle de 20 à 120°C.

22. Procédé suivant l'une quelconque des revendications 1 à 21, dans lequel un initiateur est présent pour la réaction entre la phase aqueuse contenant du cobalt, le gaz de synthèse et le solvant organique.

23. Procédé suivant la revendication 22, dans lequel l'initiateur consiste en hydrocobalt-carbonyle et dicobalt-octacarbonyle dans le solvant organique.

24. Procédé suivant la revendication 22 ou la revendication 23, dans lequel l'initiateur consiste en cobalt-carbonyle dans la phase aqueuse.

25. Procédé suivant l'une quelconque des revendications 1 à 21, dans lequel aucun initiateur n'est présent pour la réaction entre la phase aqueuse contenant du cobalt, le gaz de synthèse et le solvant organique.

26. Procédé suivant l'une quelconque des revendications 1 à 25, dans lequel des composés de cobalt volatils sont recueillis par extraction.

27. Procédé suivant la revendication 26, dans lequel l'extraction est effectuée au moyen de gaz de synthèse.

28. Procédé suivant la revendication 27, dans lequel le gaz de synthèse contenant du cobalt est mis en contact avec une charge oléfinique amenée à un réacteur oxo, les composés de cobalt de valeur présents dans le gaz se dissolvant ou étant entraînés dans la charge oléfinique.

29. Procédé suivant l'une quelconque des revendications 1 à 28, qui est mis en oeuvre sur une phase organique liquide qui est le produit d'une pluralité de réactions oxo catalysées par le cobalt.

FIG.1
PREFORMING IN OUTBOARD MODE

FIG. 2
AIRLESS DEMET
AND
OUTBOARD PREF

EP 0 642 488 B1